# EUROPEAN PATENT APPLICATION

(11) **EP 3 659 607 A1**
(43) Date of publication of application: **03.06.2020**
(21) Application number: 19206063.0
(22) Date of filing: 04.05.2016
(51) Int. Cl.: A61K 31/56, A61K 47/30, A61K 31/60, C08G 18/62, A61K 9/08, A61K 9/107

(54) **COMPOSITIONS AND METHODS FOR DELIVERING THERAPEUTIC AGENTS INTO THE COLON**

(30) Priority: 04.05.2015 US 201562156682 P
(62) Divisional of application: 16789978.0
(71) Applicant: The Board of Trustees of the Leland Stanford Junior University, Stanford, CA 94305-2038 (US)
(72) Inventor: SINHA, Sidhartha Ranjit, STANFORD, CA California 94305-2038 (US); RAJADAS, Jayakumar, STANFORD, CA California 94305-2038 (US); HABTEZION, Aida, STANFORD, CA California 94305-2038 (US); PAMNANI, Ravinder D., STANFORD, CA California 94305-2038 (US)
(74) Representative: Cabinet Laurent & Charras

(57) **Abstract**

Enema compositions containing corticosteroids or salicylic acid derivatives are disclosed. The compositions are liquid at room temperature but turn to gels at body temperature. They are useful for treating inflammatory bowel disease.

## Description

### FEDERALLY SPONSORED RESEARCH

This invention was made with Government support under contract DK007056 awarded by the National Institutes of Health. The Government has certain rights in the invention.

### FIELD OF THE INVENTION

The invention relates to enema compositions containing corticosteroids or salicylic acid derivatives. The compositions are liquid at room temperature but turn to gels at body temperature. They are useful for treating inflammatory bowel disease.

### BACKGROUND OF THE INVENTION

Inflammatory bowel disease (IBD) is a chronic, incurable disorder thought to be due to an exaggerated immune response to intestinal microbes in a genetically pre-disposed individual. About 1.5 million Americans suffer from inflammatory bowel disease (IBD) - either Crohn's disease (CD) or ulcerative colitis (UC) - and the prevalence is increasing globally. Both diseases are lifelong, relapsing illnesses that require induction followed by maintenance therapy. Patients with IBD often suffer many debilitating symptoms, such as bloody diarrhea and abdominal pain, and are at increased risk of colorectal cancer, particularly with uncontrolled disease.

Systemic and topical (applied directly to colonic mucosa) therapies exist. Current systemic therapies such as anti-TNF agents, immunomodulators, and steroids, while effective, have many known and potential side effects including increased risk of infections and malignancies. Topical therapies delivered rectally are recommended first line treatments, given their efficacy and fewer side effects when compared to systemic drugs. Topical therapies are effective in treating acute flares as well as maintaining remission for many patients. In fact, about half of IBD patients may benefit from topical therapy alone, since 66% of UC patients and 14% of CD patients have disease only in the distal colon and rectum, respectively.

The mainstay of topical therapy is the enema. Despite its advantages, patients with active distal colitis (those who would benefit most) are often unable to tolerate enemas due to urgency/spasm and the associated inability to retain a liquid solution for the recommended dosing and time frame (once or twice a day, retained for 1-3 hours). As a carrier for therapeutic agents, liquid enemas are effective at reaching all areas of the colon where inflammation may be present. However, liquid enemas are the most difficult for the patient to retain over time, given the near immediate urge to have a bowel movement once the liquid is present. Retention of the fluid is important in order to maximize the time for the therapeutic agent to diffuse from the carrier enema fluid to the mucosal wall of the digestive tract. The time span typically required is several hours, while the urge to evacuate one's bowels during a liquid enema is usually immediate (5-10 minutes). Alternatives to enemas for topical therapy include foam or suppository preparations. These options tend to be easier to retain but have the marked disadvantage of being unable to reach proximal areas of the left colon that are accessible with an enema. Despite the effectiveness of topical therapies, adherence remains extremely low due to inconvenience and discomfort associated with existing options. Current treatments for patients with IBD, while certainly beneficial, leave significant opportunity for improvement, particularly for topical therapies that are as effective as enemas for colitis involving the proximal colon, but are more tolerable and maintain low systemic side effects.

### SUMMARY OF THE INVENTION

In one aspect, the invention relates to an enema composition comprising:
a. a non-ionic block copolymer or mixture of polymers consisting of polyethylene glycol and polypropylene glycol blocks;
b. a phospholipid or mixture of phospholipids;
c. a corticosteroid; and
d. water;
wherein the concentration of the non-ionic block copolymer or mixture of polymers is from 200 to 400 g/L; the concentration of the phospholipid or mixture of phospholipids is from 0.04 to 4 g/L; the concentration of the corticosteroid is from 0.05 to 5 g/L (all based on total composition); and the remainder of the volume comprises water;
such that a gel comprising components a-d exhibits a gel transition temperature between 32 and 38 degrees C.

In a second aspect, the invention relates to enema composition comprising:
a. a non-ionic block copolymer or mixture of polymers consisting of polyethylene glycol and polypropylene glycol blocks;
b. a phospholipid or mixture of phospholipids;
c. a salicylic acid derivative; and
d. water;
wherein the concentration of said non-ionic block copolymer or mixture of polymers is from 100 to 300 g/L of said composition; the concentration of said phospholipid or mixture of phospholipids is from 4 to 40 g/L; the concentration of said salicylic acid derivative is from 50 to 100 g/L; and the remainder of the volume comprises water;
such that a gel comprising said components a-d exhibits a gel transition temperature between 32 and 38 degrees C.

In another aspect, the invention relates to a method for treating inflammatory bowel disease comprising administering an enema composition as described above.

### DETAILED DESCRIPTION OF THE INVENTION

Given the known issues with retention of liquid enemas and the limitations of suppositories and foams, we have developed a thermosensitive enema composition that has the advantages of liquid enema (more proximal delivery) but without retention issues associated with liquids. Briefly, this is accomplished by a composition that is a liquid at cooler temperatures, near room temperature (23°C), and then transitions into a viscous gel at closer to body temperature (37°C). One component of the composition is a non-ionic surfactant copolymer consisting of hydrophilic polyethylene glycol and hydrophobic polypropylene glycol blocks. As temperature increases, the copolymers form micelles and at higher temperatures, a polymer gel matrix is formed.

The enema composition described above in some embodiments contains either a triblock copolymer of polyethylene glycol and polypropylene glycol having a polyoxypropylene molecular mass of about 4,000 g/mol and about 70% polyoxyethylene content or having a polyoxypropylene molecular mass of about 1,800 g/mol and about 80% polyoxyethylene content. Commercially available examples of such PEO/PPO block copolymers are

| Poloxamer | Average Molecular Weight | Weight %Oxyethylene | Unsaturation,mEq/g |
|---|---|---|---|
| 124 | 2090to 2360 | 46.7±1.9 | 0.020±0.008 |
| 188 | 7680to 9510 | 81.8±1.9 | 0.026±0.008 |
| 237 | 6840to 8830 | 72.4±1.9 | 0.034±0.008 |
| 338 | 12700to 17400 | 83.1±1.7 | 0.031±0.008 |
| 407 | 9840to 14600 | 73.2±1.7 | 0.048±0.017 |

The commercially available materials may additionally contain a suitable antioxidant. The foregoing chart is taken from *http:*//*www.newdruginfo.com*/*pharmacopeia*/*usp28*/*v28230*/*usp28nf23s0_m66210.htm*. Because the copolymers are complex mixtures, they are usually described as having an average molecular weight of "about" x or a molecular mass of "about" y g/mol or a PEG content of "about" 70%. Persons of skill in the art recognize that greater precision is simply not practical, and the use of the term "about" in this context is not indefinite. If a numerical value must be assigned, most persons of skill would probably be comfortable (based on the figures above) with ± 40% on the average molecular weight or molecular mass, and ± 5% on the percentage of monomer content. Poloxamer 407 is a triblock (PEO-PPO-PEO) copolymer with an average molecular weight of about 12.5 KDa and a PEO/PPO ratio of about 2:1 (Bohorquez et al., J Colloid Interface Sci 1999; 216:34-40).

In the most convenient embodiments, i.e. those not requiring refrigeration, the lower practical limit for a transition temperature is about 25°C, with other transition temperatures of 26°C, 27°C, 28°C, 29°C, 30°C, 31°C, 32°C, 33°C, 34°C, 35°C or 36°C being possible. Optimally, the composition transitions to a viscous gel near 37° C. The transition temperature for gel formation appears to be somewhat dependent on the measurement system used to determine it. Oscillatory measurement (e.g. HAAKE RheoStress 6000, Thermo Fisher Scientific) provides a slightly lower temperature and sharper curve than rotational methods, because rotational testing can raise the apparent transition temperature if the rotation disrupts the gel formation. We have used a parallel plate rheometer for our studies, but the temperatures defining the gel transition for the purpose of the claims should be determined by oscillatory measurement. The sol-gel transition temperature is determined by making oscillation measurements at 1 rad.s-1 while the temperature is increased at 2°C.min-1. The sol-gel transition temperature is determined by plotting the storage modulus (G') as a function of temperature. For the purpose of the instant invention, we will define the gel transition temperature as the temperature at which the slope of a curve of storage modulus versus temperature, measured at 1 Hz, exceeds 5 kPa per degree Celsius.

In some embodiments, the enema composition comprises from 100 to 400 g/L of copolymer. In other embodiments, the enema composition comprises from 100 to 300 g/L of the copolymer. In other embodiments, the enema composition comprises from 200-400 g/L, 150-250 g/L, 250-350 g/L, 100-200 g/L, 200-300 g/L, 300-400 g/L, 100-150 g/L, 150-200 g/L, 200-250 g/L, 250-300 g/L, 300-350 g/L or 350-400 g/L.

In some embodiments the phospholipid or mixture of phospholipids is one or both of dipalmitoylphosphatidylcholine (DPPC) and 1,2-distearoyl-sn-glycero-3-phosphocholine (DSPC). Preferred phospholipids are glycerophospholipids, such as phosphatidic acid, phosphatidylethanolamine, phosphatidylcholine (lecithins), phosphatidylserine, and phosphoinositides. Phosphatidylcholines are particularly preferred.

In some embodiments, the corticosteroid is chosen from budesonide, dexamethasone, hydrocortisone, methylprednisolone, prednisolone, and prednisone. In some the corticosteroid is chosen from budesonide and hydrocortisone. In the examples below, the corticosteroid is budesonide.

In an embodiment, the enema composition is administered at less than 30 degrees C. In another embodiment, the enema composition is administered at less than 25 degrees C.

In a very specific embodiment, the enema composition consists essentially of:
a. from 250 to 350g/L of a triblock copolymer of polyethylene glycol and polypropylene glycol having a polyoxypropylene molecular mass of about 4,000 g/mol and about 70% polyoxyethylene content.;
b. from 0.1 to 1 g/L of a 1:1 mixture of dipalmitoylphosphatidylcholine (DPPC) and 1,2-distearoyl-sn-glycero-3-phosphocholine (DSPC);
c. from 0.05 to 0.2 g/L of budesonide; and
d. the remainder, water.

In some embodiments, the salicylic acid derivative is chosen from mesalazine, sulfasalazine, olsalazine, and balsalazide. Mesalazine is preferred. Mesalazine (INN, BAN) is also known as mesalamine (USAN) or 5-aminosalicylic acid (5-ASA).

In a very specific embodiment, the enema composition consists essentially of:
a. from 150 to 250 g/L of a triblock copolymer of polyethylene glycol and polypropylene glycol having a polyoxypropylene molecular mass of about 4,000 g/mol and about 70% polyoxyethylene content.;
b. from 5 to 20 g/L of a 1:1 mixture of dipalmitoylphosphatidylcholine (DPPC) and 1,2-distearoyl-sn-glycero-3-phosphocholine (DSPC);
c. from 60 to 80 g/L of mesalazine; and
d. the remainder, water.

Since the compositions of the invention are gels, they are free of propellants (e.g. hydrocarbon gases) and free of ingredients that are necessary for foams but unnecessary for gels. Typically the compositions described herein are free of foam stabilizers and foaming agents such as sodium lauryl sulphate, lauric acid, myristic acid, palmitic acid, stearic acid, coconut oil, carrageenan, stearic monoethonolamine, gum tragacanth, alginate, gelatin, sodium CMC, polyvinyl glycol, glycerol, sorbitol, hydrogenated castor oil, polysorbate 20, cocamidopropyl betaine, and caprylic/ capric glycerides. The greater comfort and the ability to accommodate larger volume compared to other modalities may additionally provide for a less frequent dosing schedule.

To test the ability of the enema compositions to be retained in the colon, 3% methylene blue was added to a formulation as described herein, and enemas were administered to C57BL/6 mice. Compositions embodying the invention containing methylene blue were clearly better retained than methylene blue in conventional liquid enema solutions.

A composition with budesonide (referenced as "BPL" for budesonide, polymer, lipid) was prepared according to the invention: Contents: Budesonide 10mg (0.1mg/mL), Poloxamer 407 30g (30% solution), DSPC 20mg (0.2 mg/mL) and DPPC 20mg (0.2 mg/mL). Method: DSPC (1,2-Distearoyl-sn-glycero-3-phosphocholine) (20 mg), DPPC (1,2-dipalmitoyl-sn-glycero-3-phosphocholine)(20mg) and budesonide (10mg) were dissolved in 5 mL of ethanol in a round bottom flask. The solvent was evaporated in a rotary evaporator to form a thin film in the inner surface of flask. The film was suspended in water (20 mL) and sonicated for 30 minutes to obtain a liposome solution. To this 30 g of Poloxamer was added and the volume was made up to 100mL with water at 4°C. This was stirred for 30 minutes, the trapped air bubbles were removed by centrifuging, and the stirring was continued till a homogeneous solution was obtained. Budesonide was dissolved in enough solution to obtain a 0.1mg/mL concentration.

A composition with mesalazine (referenced as "MPL" for mesalazine, polymer, lipid) was also prepared. Method: Phospholipids 1,2-Distearoyl-sn-glycero-3-phosphocholine (DSPC) (50 mg), (CordenPharma), 1,2-Dipalmitoyl-sn-glycero-3-phosphocholine (DPPC) (50 mg), (CordenPharma), and mesalazine (667 mg), (AK Scientific), were dissolved in 30 mL of ethanol in a round bottom flask. The ethanol was evaporated in a rotary evaporator to form a thin film in the inner surface of a flask. The film was suspended in water (3.5 mL) and sonicated for 30 minutes to obtain a homogeneous liposomal solution. A solution containing 2g of poloxamer in 5 mL of water was then added to the liposomes at 4°C. The mixture was stirred for 30 minutes and the trapped air bubbles were removed by centrifuging at 600 × g in Heraeus Labofuge - 400 centrifuge. Water (∼1 mL) was added at 4°C to make the final volume 10mL to obtain the concentration 67 mg/mL. The stirring was continued at 4°C until a homogeneous solution (mesalazine-poloxamer-lipid, MPL) was obtained. The final solution contained mesalazine (67 mg/mL), phospholipids (10 mg/mL) and poloxamer (20% w/v) in water.

Phospholipids 1,2-Distearoyl-sn-glycero-3-phosphocholine (DSPC) (50 mg), (CordenPharma), 1,2-Dipalmitoyl-sn-glycero-3-phosphocholine (DPPC) (50 mg), (CordenPharma), and mesalazine (667 mg), (AK Scientific), were dissolved in 30 mL of ethanol in a round bottom flask. The ethanol was evaporated in a rotary evaporator to form a thin film in the inner surface of a flask. The film was suspended in water (3.5 mL) and sonicated for 30 minutes to obtain a homogeneous liposomal solution. A solution containing 2g of poloxamer in 5 mL of water was then added to the liposomes at 4°C. The mixture was stirred for 30 minutes and the trapped air bubbles were removed by centrifuging at 600 × g in Heraeus Labofuge - 400 centrifuge. Water (∼1 mL) was added at 4°C to make the final volume 10mL to obtain the concentration 67 mg/mL. The stirring was continued at 4°C until a homogeneous solution (mesalazine-poloxamer-lipid, MPL) was obtained. The final solution contained mesalazine (67 mg/mL), phospholipids (10 mg/mL) and poloxamer (20% w/v) in water.

Another mesalazine composition with 6.6% mesalazine in 60 mL total volume was also prepared. Method: Ten grams of Poloxamer 407 (0.79 mmol) was melted at 60°C neat and 0.3 g of DSPC (0.77 mmol) was added followed by 0.3 g of DPPC (0.4 mmol). The mixture was stirred and 4 g of 5-aminosalicylic acid (26.12 mmol) was added. The mixture was stirred with mechanical stirring for approximately 1 hour, then cooled to 4°C. Sixty mL of deionized water was added and the mixture was stirred at 4°C overnight. The resulting liquid exhibited a transition temperature of 32-33°C.

The foregoing formulation can be expanded to a 100 mL total volume with the same mass of total drug (4g for mesalazine, 6 mg for budesonide). The concentrations are, of course, lower in the 100-mL volume solutions, but are still effective. For mesalazine, the concentration would be 40 mg/mL, and for budesonide, this would be 0.06 mg/mL.

We conducted animal studies of BPL in a well-established colitis model, dextran sulphate sodium (DSS) (orally administered) model, in C57BL/6 mice. DSS was added to the drinking water and given daily starting on Day 0. The mice were given enema treatments on Day 4 and Day 6. Daily weights were recorded, as weight is one of the most commonly used metrics to determine treatment response in colitis models. The BPL group demonstrated improvement versus water and polymer controls, and also over budesonide liquid (BL), as evidenced by less weight loss compared to original body weight. These results were replicated in two additional experiments in a total of fifty mice. In addition to weight, we also measured colon length after treatment for all mice. Mice treated with BPL had longer colons (with well formed stool pellets) compared to other treatment and control groups. Histopathology of sections of explanted mice colons showed reduced leukocyte infiltrate and more preserved epithelial architecture in BPL mice compared to other groups.

Next we conducted experiments to establish the retention kinetics and proximal distribution of BPL compared to a standard liquid treatment enema (BL). To confirm that enemas with BPL will have similar proximal distribution as BL, we gave standardized enemas with barium contrast to both healthy and DSS-induced colitis mice. The mice were then imaged with computerized tomography at pre-determined intervals (at 0.5, 1.5, and 3 hours) to evaluate maximum retrograde distance reached by the enema. BPL was shown to actually have better retrograde distribution than BL in both healthy mice and mice with colitis. In addition, in order to determine the possible mucoadhesiveness of BPL versus BL we utilized 3-D imaging software to assess the volume of enema retained at the same pre-determined intervals. Again, the volume of enema retained at all time points measured was substantially greater for BPL than BL in both healthy mice and mice with colitis.

Similar to the experiments with BPL, the MPL formulation demonstrated improvement versus water and polymer controls and also over mesalazine liquid (ML) as evidenced by less weight loss compared to original body weight. These results were replicated in an additional experiment with a total of 33 mice. Mice treated with MPL had longer colons compared to other treatment and control groups. Histopathology of sections of explanted mice colons showed healthier mucosa in MPL mice compared to other groups.

To further test the robustness of the therapy in treating colitis, another commonly used IBD colitis model--trinitrobenzene sulfonic acid (TNBS)-induced acute colitis model-was also used to test the compositions described herein in Balb/c mice. We tested BPL vs. BL and water control. Again, the BPL group regained weight faster than the other two groups after receiving TNBS enema followed by a single treatment or water enema. This experiment was repeated with consistent results.

It should be understood that in addition to the ingredients particularly mentioned above, the formulations of this invention may include other agents conventional in the art of pharmaceutical gels.

As used herein, the terms "treatment" or "treating," or "palliating" or "ameliorating" refer to an approach for obtaining beneficial or desired results including but not limited to therapeutic benefit and/or a prophylactic benefit. A therapeutic benefit is achieved with the eradication or amelioration of one or more of the physiological symptoms associated with the underlying disorder such that an improvement is observed in the patient, notwithstanding that the patient may still be afflicted with the underlying disorder. The compositions may be administered to a patient reporting one or more of the physiological systems of Inflammatory Bowel Disease, even though a diagnosis of this disease may not have been made.

### Materials and Methods

C57BL/6 mice were purchased from Taconic (Hudson, NY) and housed for one week before experiments. Balb/c mice were purchased from Jackson Laboratory (Bar Harbor, ME) and bred in house for TNBS colitis studies. Care and use of animals were in accordance to National Institutes of Health guidelines and approved by Stanford University institutional animal care and use committees.

The shear modulus and viscosity of copolymer thermogels were measured with an Ares® rheometer (Rheometric Scientific®) equipped with an oven for precise temperature control in a parallel plate geometry. Frequency scan measurements (0.5 - 200 rad/s) were performed for every temperature, while maintaining a constant strain of 1%.

C57BL/6 mice were given 2% (w/v) dextran sulfate sodium salt (36,000-50,000 M.W.; MP Biomedicals, LLC; Solon, OH) in drinking water for the duration of the experiment. Mice were weighed daily and assessed for presence of bloody stool, diarrhea and general well being. Treatment (BPL, BL) and control (water, polymer only) enemas were given on day 4 and day 6. Isoflorane-anesthetized mice were rectally given 150 µL of indicated solution through a 1 mL Luer Lock syringe attached to 23G needle and polyethylene tubing (0.048 O.D. in), with tubing inserted ∼2 cm. Animals were sacrificed on day 9 or earlier if moribund or their weight loss exceeded 20%.

Balb/c mice were anesthetized with ketamine and rectally treated with 2 mg picrylsulfonic acid (2,4,6-trinitrobenzenesulfonic acid solution; Sigma-Aldrich; St. Louis, MO) in 100 µL volume, as previously described to induce TNBS colitis. Control mice received 100 µL of vehicle (40% ethanol). The next day (day 1) mice were anesthetized with isoflurane and given treatment or control enemas of 150 µL, as with DSS colitis model. Animals were weighed and assessed daily and sacrificed at or before loss of 20% body weight.

For histological assessment, the most-distal 1 cm colon segments were fixed in 10% formalin-buffered, paraffin-embedded and sectioned for hematoxylin and eosin staining. Histopathology was scored in blinded fashion as described by Ostanin et al. "T cell transfer model of chronic colitis: concepts, considerations, and tricks of the trade." Am. J. Physiology. Gastrointestinal and liver physiology 296, G135-146 (2009*).*

Statistical analyses were done using Prism software (GraphPad Software; San Diego, CA) and statistical tests employed are indicated in the figures, with p-values of <0.05 considered as significant.

For CT imaging C57BL/6 mice were given water containing 2% (w/v) DSS and on day 6 (when bloody stool is apparent) were administered 150 µL BPL or BL enemas containing 5% (v/v) barium sulfate suspension (E-Z-EM, Inc.; Lake Success, NY). The enema tubing was inserted in a standardized manner with insertion between 0.5 to 1.0 cm. The animals were imaged in the Inveon PET-CT with large field CT scanner (Siemens Medical Solutions USA, Inc.; Hoffman Estates, IL) at the Stanford Center for Innovation in In-Vivo Imaging (Stanford, CA). Retrograde distance and enema volume were calculated.

Animal studies suggest that topically applied inhibitors of tumor necrosis factor (TNF)-α ameliorate colitis severity in animal models. Suitable concentrations of (TNF)-α could be formulated analogously to those for budesonide and mesalazine.

A first aspect (I) of the invention concerns an enema composition comprising:
a. a non-ionic block copolymer or mixture of polymers consisting of polyethylene glycol and polypropylene glycol blocks;
b. a phospholipid or mixture of phospholipids;
c. a corticosteroid; and
d. water;
wherein the concentration of said non-ionic block copolymer or mixture of polymers is from 200 to 400 g/L; the concentration of said phospholipid or mixture of phospholipids is from 0.04 to 4 g/L; the concentration of said corticosteroid is from 0.05 to 5 g/L; and the remainder of the volume comprises water;
such that a gel comprising said components a-d exhibits a gel transition temperature between 32 and 38 degrees C.

In relation with the first aspect (I) of the invention, five different preferred embodiments are described herein below.

According to a first preferred embodiment of the first aspect (I) of the invention, at least one said block copolymer is triblock copolymer of polyethylene glycol and polypropylene glycol having a polyoxypropylene molecular mass of about 4,000 g/mol and about 70% polyoxyethylene content.

According to a second preferred embodiment of the first aspect (I) of the invention, at least one said block copolymer is triblock copolymer of polyethylene glycol and polypropylene glycol having a polyoxypropylene molecular mass of about 1,800 g/mol and about 80% polyoxyethylene content.

According to a third preferred embodiment of the first aspect (I) of the invention, the enema composition comprises from 250 to 350g/L of said block copolymer.

According to a fourth preferred embodiment of the first aspect (I) of the invention, phospholipid or mixture of phospholipids is one or both of dipalmitoylphosphatidylcholine (DPPC) and 1,2-distearoyl-sn-glycero-3-phosphocholine (DSPC).

According to a fifth preferred embodiment of the first aspect (I) of the invention, said corticosteroid is chosen from budesonide, dexamethasone, hydrocortisone, methylprednisolone, prednisolone, and prednisone.

In relation with the above fifth preferred embodiment of the first aspect (I) of the invention, said corticosteroid is chosen from budesonide and hydrocortisone.

Furthermore and in relation with the above fifth preferred embodiment of the first aspect (I) of the invention, said corticosteroid is budesonide.

Furthermore and in relation with the above fifth preferred embodiment of the first aspect (I) of the invention, the enema composition consists of:
a. from 250 to 350g/L of a triblock copolymer of polyethylene glycol and polypropylene glycol having a polyoxypropylene molecular mass of about 4,000 g/mol and about 70% polyoxyethylene content.;
b. from 0.1 to 1 g/L of a 1:1 mixture of dipalmitoylphosphatidylcholine (DPPC) and 1,2-distearoyl-sn-glycero-3-phosphocholine (DSPC);
c. from 0.05 to 0.2 g/L of budesonide; and
d. the remainder, water.

Furthermore and in relation with the above fifth preferred embodiment of the first aspect (I) of the invention, the enema composition is of 60 mL to 100 mL total volume.

A second aspect (II) of the invention concerns an enema composition comprising:
a. a non-ionic block copolymer or mixture of polymers consisting of polyethylene glycol and polypropylene glycol blocks;
b. a phospholipid or mixture of phospholipids;
c. a salicylic acid derivative; and
d. water;
wherein the concentration of said non-ionic block copolymer or mixture of polymers is from 100 to 300 g/L; the concentration of said phospholipid or mixture of phospholipids is from 4 to 40 g/L; the concentration of said salicylic acid derivative is from 50 to 100 g/L; and the remainder of the volume comprises water;
such that a gel comprising said components a-d exhibits a gel transition temperature between 32 and 38 degrees C.

In relation with the second aspect (II) of the invention, five different preferred embodiments are described herein below.

According to a first preferred embodiment of the second aspect (II) of the invention, at least one said block copolymer is triblock copolymer of polyethylene glycol and polypropylene glycol having a polyoxypropylene molecular mass of about 4,000 g/mol and about 70% polyoxyethylene content.

According to a second preferred embodiment of the second aspect (II) of the invention, at least one said block copolymer is triblock copolymer of polyethylene glycol and polypropylene glycol having a polyoxypropylene molecular mass of about 1,800 g/mol and about 80% polyoxyethylene content.

According to a third preferred embodiment of the second aspect (II) of the invention, the enema composition comprises from 150 to 250 g/L of said block copolymer.

According to a fourth preferred embodiment of the second aspect (II) of the invention, phospholipid or mixture of phospholipids is one or both of dipalmitoylphosphatidylcholine (DPPC) and 1,2-distearoyl-sn-glycero-3-phosphocholine (DSPC).

According to a fifth preferred embodiment of the second aspect (II) of the invention, said salicylic acid derivative is chosen from mesalazine, sulfasalazine, olsalazine, and balsalazide.

In relation with the above fifth preferred embodiment of the second aspect (II) of the invention, said salicylic acid derivative is mesalazine.

Furthermore and in relation with the fifth preferred embodiment, the enema composition consists of:
a. from 150 to 250 g/L of a triblock copolymer of polyethylene glycol and polypropylene glycol having a polyoxypropylene molecular mass of about 4,000 g/mol and about 70% polyoxyethylene content.;
b. from 5 to 20 g/L of a 1:1 mixture of dipalmitoylphosphatidylcholine (DPPC) and 1,2-distearoyl-sn-glycero-3-phosphocholine (DSPC);
c. from 60 to 80 g/L of mesalazine; and
d. the remainder, water.

Furthermore and in relation with the above fifth preferred embodiment of the second aspect (II) of the invention, the enema composition is of 60 mL to 100 mL total volume.

A third aspect (III) of the invention concerns a method of treating inflammatory bowel disease comprising administering:
an enema composition comprising:
   a. a non-ionic block copolymer or mixture of polymers consisting of polyethylene glycol and polypropylene glycol blocks;
   b. a phospholipid or mixture of phospholipids;
   c. a corticosteroid; and
   d. water;
wherein the concentration of said non-ionic block copolymer or mixture of polymers is from 200 to 400 g/L; the concentration of said phospholipid or mixture of phospholipids is from 0.04 to 4 g/L; the concentration of said corticosteroid is from 0.05 to 5 g/L; and the remainder of the volume comprises water;
such that a gel comprising said components a-d exhibits a gel transition temperature between 32 and 38 degrees C ;
   or
an enema composition comprising a non-ionic block copolymer or mixture of polymers consisting of polyethylene glycol and polypropylene glycol blocks;
   b. a phospholipid or mixture of phospholipids;
   c. a salicylic acid derivative; and
   d. water;
wherein the concentration of said non-ionic block copolymer or mixture of polymers is from 100 to 300 g/L; the concentration of said phospholipid or mixture of phospholipids is from 4 to 40 g/L; the concentration of said salicylic acid derivative is from 50 to 100 g/L; and the remainder of the volume comprises water;
such that a gel comprising said components a-d exhibits a gel transition temperature between 32 and 38 degrees C.
to a patient in need of such treatment.

In relation with the third aspect (III) of the invention, two different preferred embodiments are described herein below.

According to a first preferred embodiment of the third aspect (III) of the invention, the enema composition is administered at a temperature below the gel temperature of the composition.

According to a second preferred embodiment of the third aspect (III) of the invention, the enema composition is at less than 30 degrees C when administered.

Although this invention is susceptible to embodiment in many different forms, preferred embodiments of the invention are shown. It should be understood, however, that the present disclosure is to be considered as an exemplification of the principles of this invention and is not intended to limit the invention to the embodiments illustrated.

## Claims

1. A method of making an enema composition, the method comprising:
a) producing a dried composition comprising one or more phospholipids and a salicylic acid derivative; and
b) introducing a solution comprising a non-ionic block copolymer or mixture of polymers consisting of polyethylene glycol and polypropylene glycol blocks, thereby forming the enema composition.

2. The method of claim 1, wherein the salicylic acid derivative is mesalamine.

3. The method of claim 1 or 2, wherein the one or more phospholipids is one or more of dipalmitoylphosphatidylcholine (DPPC), 1,2-distearoyl-sn-glycero-3-phosphocholine (DSPC), or phosphatidylcholines (lecithins).

4. The method of any one of claims 1-3, wherein the produced enema composition is a liquid at room temperature.

5. The method of any one of claims 1-4, wherein the produced enema composition exhibits a gel transition temperature from about 25 degrees C to about 38 degrees C.

6. The method of any one of claims 1-5, wherein the non-ionic block copolymer or mixture of polymers is a poloxamer.

7. The method of claim 6, wherein the poloxamer is poloxamer 407.

8. The method of any one of claims 1-7, wherein the solution further comprises water.

9. The method of any one of claims 1-8, wherein the method excludes addition of propellants and ingredients necessary for foams such that a person is able to accommodate larger volumes of the enema composition as compared to other modalities, allowing for a less frequent dosing schedule.

10. The method of any one of claims 1-9, wherein the enema composition comprises a gel transition temperature that exceeds 5 kPa per degree Celsius.
